(19) Europäisches Patentamt
European Patent Office
Office européen des brevets

(11) **EP 3 398 508 A1**

(12) **EUROPEAN PATENT APPLICATION**

(43) Date of publication:
**07.11.2018 Bulletin 2018/45**

(21) Application number: **17169542.2**

(22) Date of filing: **04.05.2017**

(51) Int Cl.:
**A61B 5/024** (2006.01)　　　**A61B 5/00** (2006.01)
**G06T 7/00** (2017.01)　　　**A61B 5/1455** (2006.01)
**A61B 5/1171** (2016.01)　　　**A61B 5/08** (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Koninklijke Philips N.V.
5656 AE Eindhoven (NL)**

(72) Inventors:
• **BULUT, Murtaza
5656 AE Eindhoven (NL)**

• **JEANNE, Vincent
5656 AE Eindhoven (NL)**
• **ASSELMAN, Michel Jozef Agnes
5656 AE Eindhoven (NL)**
• **KERSTEN, Gerrit Maria
5656 AE Eindhoven (NL)**
• **DEN BRINKER, Albertus Cornelis
5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **A SYSTEM AND METHOD FOR EXTRACTING A PHYSIOLOGICAL INFORMATION FROM VIDEO SEQUENCES**

(57) There is provided a method of extracting a physiological information which comprises, for a defined time-period, extracting a plurality of signal segments in a time-domain form, selecting a subset of the plurality of signal segments according their maximum signal level, converting at least said subset to produce a plurality of transformed signal segments, and combining the at least said subset of the plurality of transformed signal segments to produce a combined signal segment. This has the advantage of reducing the influence of motion and or illumination-induced changes on the final result by removing parts of the raw signal segments which are worst affected by these.

Fig 4

EP 3 398 508 A1

**Description**

FIELD OF THE INVENTION

The present invention relates to the extraction of information from video sequences and, in particular to the extraction of physiologically-related information (often referred to as vital signs).

BACKGROUND OF THE INVENTION

It is possible to analyze video sequences of a living subject and detect small changes in the images which are the result of physiological processes of that subject. Amongst these physiological process are such things as blood flow, breathing and sweating.

Certain physiological processes can be observed via skin reflectance variations. The human skin can be modelled as an object with at least two layers, one of those being the epidermis (a thin surface layer) and the other the dermis (a thicker layer underneath the epidermis). A certain percentage 5 % of an incoming ray of light is reflected at the skin surface. The remaining light is scattered and absorbed within the two skin layers in a phenomenon known as body reflectance (described in the Dichromatic Reflection Model). The melanin, typically present at the boundary of epidermis and dermis, behaves like an optical filter, mainly absorbing light. In the dermis, light is both scattered and absorbed. The absorption is dependent on the blood composition, so that the absorption is sensitive to blood flow variations. The dermis contains a dense network of blood vessels, about 10 % of an adult's total vessel network. These vessels contract and expand according of the blood flow in the body. They consequently change the structures of the dermis, which influences the reflectance of the skin layers.

Other physiological processes such as breathing cause movement in the surface of patient.

Other physiological processes such as variations in blood oxygenation level can manifest themselves as small colour changes.

It is possible to detect and extract signals which have some periodic content in these changes and from that obtain a result such as a frequency in the case of periodic processes. For example, a subject may be illuminated with ambient light and filmed using a video camera. By analyzing changes in the values of corresponding pixels between frames of the sequence of images, a time-variant signal can be extracted. This signal may be transformed into frequency-like domain using something like a Fast Fourier Transform and from the frequency-domain spectra, a value for the subject's heart-rate may be arrived at as a physiological measurement. These physiological measurements are often called vital signs.

The changes in the pixel values are often small and often more pronounced in 1 colour channel than the others. Thus the signal that is being looked for is correspondingly small.

There may be other changes in the pixel values such as those due to changes in the general image and these can be large in comparison to the signal. There are also sources of random change in the pixel values such as movement of the subject, changes in the illumination (such as flicker) and noise in the image sensor and variations in the illumination. All of these are, to all intents and purposes, uncorrelated with the signal being sought. Therefore the signal to noise ratio is small and it may be difficult to obtain a meaningful physiological measurement.

SUMMARY OF THE INVENTION

There is provided a method of extracting a physiological information comprising, for a defined time-period, extracting a plurality of signal segments in a time-domain form, selecting a subset of the plurality of signal segments according their maximum signal level, converting at least said subset to produce a plurality of transformed signal segments, and combining the at least said subset of the plurality of transformed signal segments to produce a combined signal segment.

This has the advantage of reducing the influence of motion and or illumination-induced changes on the final result by removing parts of the raw signal segments which are worst affected by these.

According to an embodiment, the method comprises rejecting segments having a maximum signal level greater than a threshold. This exploits the fact that the signal of interest which contains the physiological information is small and so large signals can be considered as being due to motion or illumination changes.

According to an embodiment, the selecting comprises weighting segments with weights having an inverse relationship to their maximum signal level. This has the advantage of not requiring a hard threshold which may be difficult to set satisfactorily.

According to an embodiment, the inverse relationship is of the form of a power law or an inverse exponential which has the advantage of providing a strong selectivity for small signal levels as compared to large signal levels.

According to an embodiment, the conversion is performed using one of a power spectral density function, an auto-correlation function or a Fourier transform which provide the signal in a frequency-like domain from which the physiological information, which is in the form of a frequency, may be extracted.

According to an embodiment, the combining of the signal segments comprises averaging the signal segments which affords a signal which had further reduced levels of unwanted components.

According to an embodiment, method further comprisises epeating at least once the method described above, storing the combined signal segment at each repetition to produce a plurality of combined signal segments, and averaging the plurality of combined signal segments to produce a final combined signal. This affords further reduction of unwanted components.

According to an embodiment, the settings for the averaging of the plurality of combined signal segments are derived from a previously stored combined signal segment. This allows for adaptive filtering or averaging which helps reduce unwanted components.

According to an embodiment, the method further comprises performing an interpolation of the combined signal which provides further smoothing an unwanted component reduction.

According to an embodiment, the method further comprises extracting a value representative of the physiological information which may then be provided as an output.

According to an embodiment, the method further comprises acquiring a sequence of video frames and the extracting of the plurality of signal segments is performed using remote photoplethysmography. This has the advantage of affording a non-contact (or non-invasive) method of acquiring the signals for analysis and may be performed using a video-camera.

There is provided a system for extracting a physiological information representative of a vital sign comprising a signal extraction unit configured to, for a defined time-period, extract a plurality of signal segments in a time-domain form, a selection unit configured to select a subset of the plurality of signal segments according their maximum signal level, a convertion unit configured to convert at least said subset to produce a plurality of transformed signal segments, and a combining unit configured to combine the at least said subset of the plurality of transformed signal segments to produce a combined signal segment.

According to an embodiment, the combining unit of the system further comprises a time-averaging unit configured to store the combined signal segments, to store interpolation settings, to apply those stored settings to interpolate subsequent combined signal segments and to combine the stored combined signal segments. This allows reduction of unwanted components by averaging over a longer period of time.

There is provided a computer program software product, stored on a computer-readable medium, configured, when executed on a processor, to execute method described herein. This allows a general purpose computer, when linked to a video camera to operate the method described herein.

There is provided a physiological measurement equipment comprising a system as described herein.

BRIEF DESCRIPTION OF THE DRAWINGS

The above, as well as additional objects, features and advantages of the disclosed systems and methods, will be better understood through the following illustrative and non-limiting detailed description of embodiments of devices and methods, with reference to the appended drawings, in which:

Fig. 1 represents a setup according to an embodiment for measuring a physiological information of a subject.
Fig. 2 represents a video sequence processing chain according to an embodiment.
Fig. 3 represents a case of performing the measurement according to an embodiment.
Fig. 4 represents a process according to an embodiment of extracting a physiological information.
Fig. 5 represents a flow of a method according to an embodiment.

DETAILED DESCRIPTION OF THE EMBODIMENTS

In the following description, same references designate like elements.

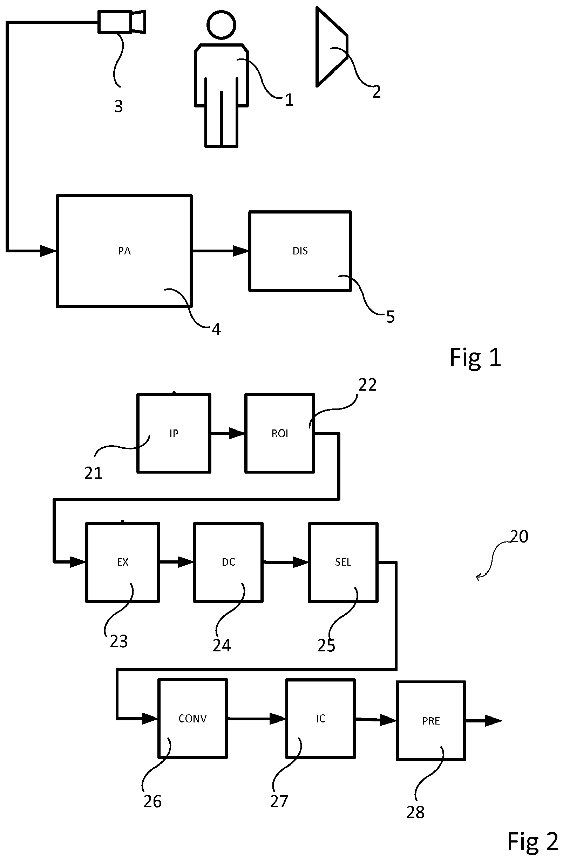

Fig. 1 represents a setup for measuring a physiological process of a subject 1 and extracting a physiological information or vital sign. A light source 2 (which may be artificial or natural) illuminates the subject 1. A video camera 3 records a sequence of video frames and feeds them to a processing unit (PA) 4 which, extracts the vital sign and in turn provides an output to a display 5. The display 5 may display just the physiological information from the signal analysis unit 4 either alone or in combination with the video sequence.

Fig. 2 represents a processing chain 20 according to an embodiment and configured to extract the physiological information. The processing chain 40 may be conveniently implemented as part of the processing device 4. An input (IP) 21 receives the video sequence and passes the frames of the video sequence to a patch selecting unit 22 (ROI) which selects the patches or ROIs in the images of the video sequence that are to be tracked. There may be one or more patches which are selected for subsequent processing. The patch selecting unit 22 feeds a series patches to a signal extractor 23 (EX). The signal extractor 23 performs operations on the signal in order to arrive at the time-varying signal of interest. These operations may include the combining of the colour channels and/or the normalizing of the signals. It may be advantageous to perform motion compensation and so it may be that the sequence of patches has been broken up into shorter sequences in order to make the task of motion compensation easier. The extracted time-varying signals are then fed to a decomposition unit (DC) 24 which performs a decomposition of the signals so as to be able to remove the DC component and perform some cleaning. The cleaning may involve removal of high-frequent noise (by low-pass filtering), removal of disturbing components (e.g. spikes by median filtering), or rejection of signals with large signal discontinuities.Then the cleaned signals are fed to a signal selection unit (SEL) 25 which selects amongst the signals those which will be used for further processing. A conversion unit (CONV) 26 performs a transformation of the time-varying signals so as to allow extraction of a periodic property of the signal, the periodic property being representative of the physiological information or vital sign. The transformed signals are then fed to a combining unit (IC) 27 which combines the transformed signals into a single signal and feeds this single signal to a physiological measurement extractor (PRE) 28 which extracts the physiological measurement from this single signal.

Thus there is method of extracting a physiological information over a time-period which comprises extracting a plurality of signal segments in a time-domain form, selecting a subset of the plurality of signal segments according their maximum signal level and then converting at least said subset to produce a plurality of transformed signal segments, and then combining the at least said subset of the plurality of transformed signal segments to produce a combined signal segment.

The processing chain 20 may be implemented in one or more general purpose processors running appropriate software. This has the advantage of being possible with pre-existing hardware and allows for subsequent modification and tuning. However it can result in a solution which is slower and/or more expensive than a mode dedicated solution. Alternatively some or all of the individual components may be implemented in microcontrollers running firmware designed to implement the relevant functions. This solution may be less expensive when production volumes are sufficiently high enough. Yet another possibility is to implement the functions in dedicated hardware. In high volumes, this is often cheaper and gives higher processing speed per unit cost.

The patches are selected using one or more of a number of methods. A process which is sometimes called 'segmentation' is performed. It is convenient to start by selecting the general area of interest. The face is suitable whenever blood flow is the physiological process of interest so a face-identification algorithm may be used. A suitable algorithm for implementing face detection is described in Viola, P. and Jones, M.J., "Robust real-time object detection", Proc. of IEEE workshop on statistical and computational theories of vision, 13 July 2001. Alternative algorithms for recognizing shape and colour patterns also exist and these may be used for detecting the facial area. For other processes like breathing, other methods for identifying the thorax may be used.

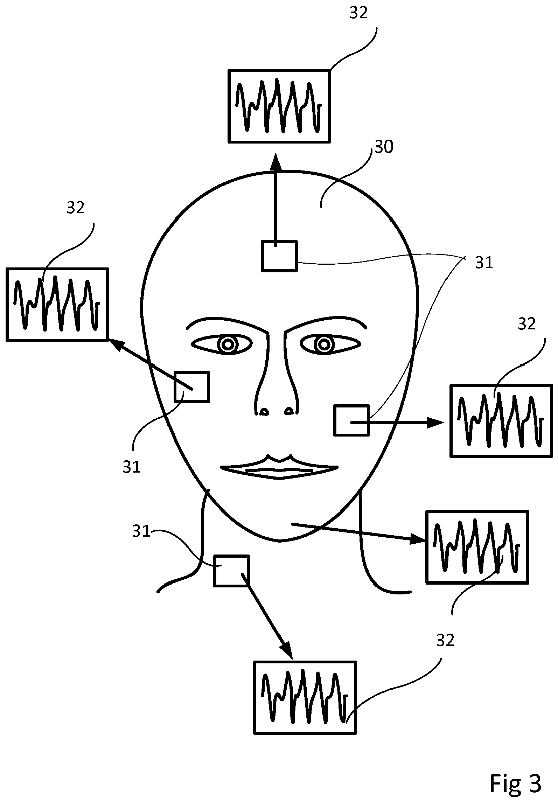

Fig. 3 represents an exemplary situation where a selected region 30 (in this case a face of a subject 1) is being used. A plurality of patches 31 has been selected from which time-varying signals 32 have been extracted over a period of time. Whilst the time-varying signals 32 have been extracted at nominally the same time, they are not perfectly synchronized. Therefore, whilst these time varying signals 32 contain the same physiological information, it is not effective to combine them in the time-domain. Also, as mentioned previously, for the purposes of motion compensation, the time-varying signal 32 from each patch 31 may be collected as a series of signal segments over a longer period of time, with the objective of combining these signal segments into a single signal.

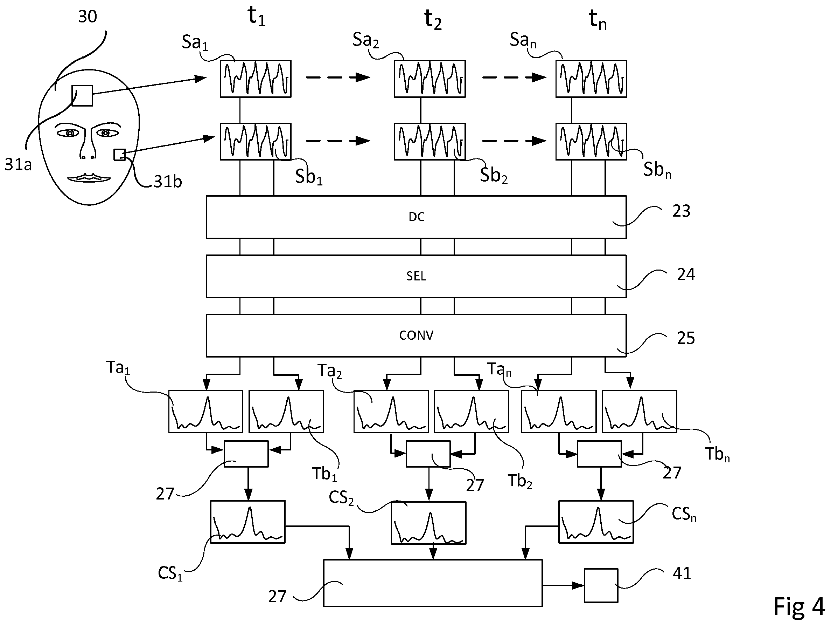

Fig. 4 represents a process or method according to an embodiment, as described above but in more detail. From a plurality of patches 31a, 31b on the selection region 30, over a series of time slots $t_1$, $t_2$, $t_3$, a plurality of series of time-varying signals segments $Sa_{1-n}$, $Sb_{1-n}$ are extracted and collected. Each of the time-varying signals $Sa1-n$, $Sb1-n$ is individually processed by a decomposition unit 24 which performs DC removal and cleaning. From the individual time-varying signals segments $Sa_{1-n}$, $Sb_{1-n}$, the selecting unit 25 selects the preferred signal segments that will ultimately combined for extraction of the physiological information.

The selection is performed so as to remove those signal segments which would degrade the overall signal-noise-ratio. The inventors have found that a significant source of problems are those signal segments that come from patches where motion of the subject 1 or variations in the illumination are observable and that these patches can be identified by the amplitude of the time-varying signal extracted therefrom. The inventors have made the surprising observation that amplitudes which are too great are indicative of the presence of problems, particularly motion or illumination-variation artifacts. This is somewhat counter intuitive in that normally in a search for greater signal-to-noise ratios, the skilled person would prefer greater signal amplitudes and reject those with smaller amplitudes. There are various embodiments for the selection process. Firstly, the patches may be assessed in a number of ways. The standard deviation of the pixel values in the patch over the time period concerned may be used because this is related to the amplitude of the time-varying signal extracted therefrom. Another possibility is to assess the total power or energy of the signal which is given by the variance of the pixel values of the patch over time. These have the advantage of being easy to integrate into the signal processing chain since such functions are also used for the motion tracking. A further possibility is to assess the maximum signal amplitude in the actual time-varying signal segments, which is more direct and may be easier to tune but requires extra functions and processing. In the case where the selection is performed by examining the pixel values of the patch, the selection could be made before signal extraction which could save some processing power.

Next the results of the assessment are used to make a selection. This can be selecting a maximum threshold value and rejecting all cases exceeding this. An alternative is to apply a weighting to the individual time-varying signal segments where the weightings have an inverse relationship to the signal amplitude (measured or inferred). It is also possible to use a relationship which is not linear but has a power law form like

$$f(x) = ax^{-k}$$

or an inverse exponential form like

$$f(x) = ae^{-bx}$$

The linear scaling factor a, the power law exponent and the exponential scaling factor may be adjusted to give the desired results. Using this weighting technique, particularly with a non-linear function has the advantage that it allows the rejection of the outlying i.e. very large values whilst reducing the difficulty of accurately determining the best parameters (which might vary between situations or equipment characteristics) in that they operate more 'softly'. Nevertheless, it is also possible to combine selection methods such as using a threshold and then a linear and/or non-linear weighting methods. And advantage of this could be computation speed in that very extreme results could be excluded without the need to calculate the weightings, thereby saving time and computational effort.

The selected time-varying signal segments $Sa_{1-n}$, $Sb_{1-n}$ are then transformed by the conversion unit 25 into transformed signal segments $Ta_{1-n}$, $Tb_{1-n}$, of a form from which a dominant frequency or periodicity can be extracted. Such operations could be transforming into a spectrum, i.e. frequency domain, by using something like a Discrete or Fast Fourier Transform (DFT or FFT). From the spectrum the DC component and other components considered out-of-band may be discarded and a peak corresponding to the fundamental frequency of the pertinent physiological process.

A DFT may be expressed as, for a sequence of N complex numbers $X_n$

$$X_k \stackrel{\text{def}}{=} \sum_{n=0}^{N-1} x_n . e^{-j2\pi kn/N}$$

Another method could be to use an autocorrelation or ACF (also sometimes known as a cross-autocorrelation or serial correlation) function to arrive at result indicative of a quasi-periodic signal. By way of illustration only, a common formation of an estimation for autocorrelation function for a signal for which n observations have been made and for which there are mean $\mu$, and variance $\sigma^2$:

$$\hat{R}(k) = \frac{1}{(n-k)\sigma^2} \sum_{t=1}^{n-k} (X_t - \mu)(X_{t+k} - \mu)$$

where k is an integer less than n.

From the inverse of the time lag between the peaks of the autocorrelation, a frequency of the periodic signal can be derived.

A third method could be to use a power spectral density function (PSDF). This function represents the frequency distribution of the power of a signal. It is sometimes defined or expressed as, for a finite time series $X_n$ of samples of a signal, the samples being at discrete times $X_n = x(n\Delta t)$ for a total time period T= $N\Delta t$:

$$S_{xx}(\omega) = \frac{(\Delta t)^2}{T} \left| \sum_{n=1}^{N} x_n \, e^{-i\omega n} \right|^2$$

where n is between 1 and N

It may be useful to vary the above expressions or choose different formulations, possibly with other terms, when implementing them in a system for extracting physiological information.

Another possibility is a Laplace transform which can also be used to obtain a frequency-domain representation of a signal from its time-domain form.

Other possibilities exist such as the multiple signal classification (MUSIC) algorithm, the pitch detection algorithm (PDA), the average magnitude different function (ADMF), the average squared mean difference function (ASDMF). There are also algorithms known as the YIN algorithm and the MPM algorithms respectively.

It may also be possible to use multiple transform methods. This would allow comparison of the results for the purposes of confirmation and judging the quality of the result. In a situation where the quality was too low, a reselection with tighter criteria (for example, a lower threshold, coefficients adjusted to give higher non-linearity) could be performed.

Next the transformed signal segments $Ta_{1-n}$, $Tb_{1-n}$ are combined by the combining unit 27 into combined signal segments $CS_{1-n}$. At this point, the combined signal segments $CS_{1-n}$ still correspond to the time periods $t_{1-n}$ from which they came, within the limits that some elimination may have occurred during the selection procedure described previously. It should be understood that the combination, which can be averaging the respective signal segments together, is now possible because the transformation has removed the relative phase information that prevented combination of the time-domain representations. This has the advantage of reducing still further unwanted components such as noise, distortions or artefacts introduced by the conversion process.

The combined signal segments $CS_{1-n}$, which have been collected overtime, are further combined into a final combined signal 41 from which the physiological information can be derived by finding a dominant frequency or periodic component. In this combining step, noise may be reduced by (weighted) averaging over combined signal segments $CSn$, collected over a period of time. This can be considered as executing an identical temporal filter per element of signal segments. This filter may be implemented as an FIR or IIR filter. FIR and IIR filters will give different results and the choice between them is made according to different constraints - for example a requirement on linear phase could lead more toward an FIR. Such a choice is within the reach of the skilled person. It is advantageous to perform interpolation to increase the resolution. Examples of suitable methods of interpolation are linear, polynomial or spline. Both steps can be combined and thus the combination unit acts as a time-averaging unit which is configured to store the combined signal segments, to store interpolation values, to store the filter states, and to combine the combined signal elements. The filter settings may be adaptively controlled. If filters with a short time constant already provide a clear indication of the physiological feature of interest (e.g., the frequency or repetition rate of the blood volume pulse) one may select and use this setup. However, if the feature of interest does not stand out sufficiently in the output signal, a larger time constant (i.e. other filter coefficients) can be chosen. The decision method may be as simple as a comparing values obtained for the feature to a threshold. For example, in case where the feature is a frequency associated with the highest peak in the Fourier transform, one may determine the peak value relative to the background level (environment) and let that serve as a control variable. Thus the filter settings are controlled by a local contrast measure within the results output by the function being used. A similar mechanism can be operated when searching for a delay in an ACF. Using a smaller time constant gives the advantage of obtaining a result more quickly (i.e., less latency) and/or with a lower computational load. Using longer time constants may be more accurate or give more stable results. The threshold may be chosen using a calibration routine when the apparatus is started up which would have the advantage of tending to give higher accuracy but requires more time and complexity in the equipment. Alternatively, the threshold could be set either by design or at manufacturing time - which would be cheaper. This process serves to further reduce unwanted components.

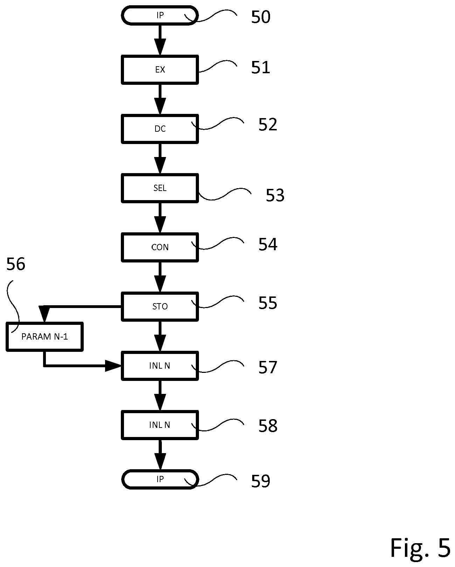

Fig. 5 represents the above described method in the form of a flow. At step 50 an input of the video sequence is received. At step 51, time varying signal segments are extracted. At step 52, the time varying signal segments are cleaned and the DC component removed. At step 53, a selection amongst the time-varying signal segments is made as described above. At step 54 the time varying signal segments are converted to a frequency or periodic representation and converted signal segments from a given time slot are combined. At step 55 the combined signal segments are stored. At step 56 interpolation parameters are derived from the combined signal segment and stored. At step 57, an interpolation of a current transformed signal segment is performed using previously stored parameters. At step 58 the interpolated stored signal segments are combined into a final signal. At step 59 a physiological information is extracted from the final signal.

It should be noted that the above-mentioned embodiments illustrate rather than limit the invention, and that those skilled in the art will be able to design many alternative embodiments without departing from the scope of the appended claims. Furthermore, in many cases, embodiments presented as alternatives may be wholly or in part combined.

In the claims, any reference signs placed between parentheses shall not be construed as limiting the claim. Use of the verb "comprise" and its conjugations does not exclude the presence of elements or steps other than those stated in a claim. The article "a" or "an" preceding an element does not exclude the presence of a plurality of such elements. The invention may be implemented by means of hardware comprising several distinct elements, and by means of a suitably programmed computer or processing unit. In the device claim enumerating several means, several of these means may be embodied by one and the same item of hardware. The mere fact that certain measures are recited in mutually different dependent claims does not indicate that a combination of these measures cannot be used to advantage.

Aspects of the invention may be implemented in a computer program product, which may be a collection of computer program instructions stored on a computer readable storage device which may be executed by a computer. The instructions of the present invention may be in any interpretable or executable code mechanism, including but not limited to scripts, interpretable programs, dynamic link libraries (DLLs) or Java classes. The instructions can be provided as complete executable programs, partial executable programs, as modifications to existing programs (e.g. updates) or extensions for existing programs (e.g. plugins). Moreover, parts of the processing of the present invention may be distributed over multiple computers or processors.

Storage media suitable for storing computer program instructions include all forms of nonvolatile memory, including but not limited to EPROM, EEPROM and flash memory devices, magnetic disks such as the internal and external hard disk drives, removable disks and CD-ROM disks. The computer program product may be distributed on such a storage medium, or may be offered for download through HTTP, FTP, email or through a server connected to a network such as the Internet.

## Claims

1. A method of extracting a physiological information comprising:

   - for a defined time-period, extracting a plurality of signal segments in a time-domain form;
   - selecting a subset of the plurality of signal segments according their maximum signal level;
   - converting at least said subset to produce a plurality of transformed signal segments, and

   combining the at least said subset of the plurality of transformed signal segments to produce a combined signal segment.

2. The method of any of the claims1 wherein selecting comprises rejecting segments having a maximum signal level greater than a threshold.

3. The method of claim 1 wherein selecting comprises weighting segments with weights having an inverse relationship to their maximum signal level.

4. The method of claim 2 wherein the inverse relationship is of the form of a power law or an inverse exponential.

5. The method of any of the previous claims wherein the conversion is performed using one of a power spectral density function, an auto-correlation function or a Fourier transform.

6. The method of any of the previous claims wherein the combining of the signal segments comprises averaging the signal segments.

7. The method of any of the previous claims further comprising:

   - repeating at least once the method of above claims;
   - storing the combined signal segment at each repetition to produce a plurality of combined signal segments, and

   averaging the plurality of combined signal segments to produce a final combined signal.

8. The method of claim 7 wherein the settings for the averaging of the plurality of combined signal segments are derived from a previously stored combined signal segment.

9. The method of claim 7 or 8 further comprising performing an interpolation of the combined signal

10. The method of any of any previous claim further comprising extracting a value representative of the physiological information.

11. The method of any previous claim further comprising the step of acquiring a sequence of video frames and wherein the extraction of the plurality of signal segments is performed using remote photoplethysmography.

12. A system for extracting a physiological information representative of a vital sign comprising:

   - a signal extraction unit configured to, for a defined time-period, extract a plurality of signal segments in a time-domain form;
   - a selection unit configured to select a subset of the plurality of signal segments according their maximum signal level;
   - a convertion unit configured to convert at least said subset to produce a plurality of transformed signal segments, and

   a combining unit configured to combine the at least said subset of the plurality of transformed signal segments to produce a combined signal segment.

13. The system of claim 10 wherein the combining unit further comprises a time-averaging unit configured to store the combined signal segments, to store interpolation settings, to apply those stored settings to interpolate subsequent combined signal segments and to combine the stored combined signal segments.

14. A computer program software product, stored on a computer-readable medium, configured, when executed on a processor, to execute method of any of claims 1 to 10.

15. A physiological measurement equipment comprising a system according to any of claims 11 to 13.

Fig 1

Fig 2

Fig 3

Fig 4

Fig. 5

Europäisches
Patentamt

European
Patent Office

Office européen
des brevets

# EUROPEAN SEARCH REPORT

**Application Number**

EP 17 16 9542

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PRATHOSH A P ET AL: "Estimation of Respiratory Pattern From Video Using Selective Ensemble Aggregation", IEEE TRANSACTIONS ON SIGNAL PROCESSING, IEEE SERVICE CENTER, NEW YORK, NY, US, vol. 65, no. 11, 2 February 2017 (2017-02-02), pages 2902-2916, XP011644307, ISSN: 1053-587X, DOI: 10.1109/TSP.2017.2664048 [retrieved on 2017-03-28] * page 2902, right-hand column, line 14 * * page 2903, right-hand column, lines 24, 25 * * page 2904, left-hand column, lines 25, 39, 41, 42 * * page 2904, right-hand column, lines 18, 19 * * page 2905, left-hand column, line 38 * * page 2906, left-hand column, lines 11, 12 * * page 2907, left-hand column, line 1 * * page 2908, left-hand column, lines 30, 31 * * page 2909, right-hand column, line 14 * * chapter "B. Real-Life Dataset"; page 2912 * * equations (1), (2), (24) * * figures 10, 11 * * abstract * | 1-15 | INV. A61B5/024 A61B5/00 G06T7/00 ADD. A61B5/1455 A61B5/1171 A61B5/08 |
| A | US 2006/293575 A1 (NORRIS MARK A [US]) 28 December 2006 (2006-12-28) * paragraph [0014] * | 4,5 | |
| A | WO 2016/193735 A1 (UNIV OXFORD INNOVATION LTD [GB]) 8 December 2016 (2016-12-08) * page 12, line 2 - line 15 * | 7,8 | |

-/--

**TECHNICAL FIELDS SEARCHED (IPC)**

A61B

The present search report has been drawn up for all claims

| Place of search | Date of completion of the search | Examiner |
|---|---|---|
| The Hague | 23 October 2017 | Meyer, Wolfgang |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

## EUROPEAN SEARCH REPORT

Application Number

EP 17 16 9542

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2006/091636 A2 (DIGITAL INTELLIGENCE L L C [US]; RICCI CARLOS A [US]; KOVTUN VLADIMIR) 31 August 2006 (2006-08-31) * paragraph [0099] * ----- | 9,13 | |
| A | WO 2014/068436 A1 (KONINKL PHILIPS NV [NL]) 8 May 2014 (2014-05-08) * the whole document * ----- | 1-15 | |
| A | WO 2015/049150 A1 (KONINKL PHILIPS NV [NL]) 9 April 2015 (2015-04-09) * the whole document * ----- | 1-15 | |

TECHNICAL FIELDS
SEARCHED      (IPC)

The present search report has been drawn up for all claims

| Place of search | Date of completion of the search | Examiner |
|---|---|---|
| The Hague | 23 October 2017 | Meyer, Wolfgang |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**

EP 17 16 9542

This annex lists the patent family members relating to the patent documents cited in the above-mentioned European search report.
The members are as contained in the European Patent Office EDP file on
The European Patent Office is in no way liable for these particulars which are merely given for the purpose of information.

23-10-2017

| Patent document cited in search report | | Publication date | Patent family member(s) | | Publication date |
|---|---|---|---|---|---|
| US 2006293575 | A1 | 28-12-2006 | NONE | | |
| WO 2016193735 | A1 | 08-12-2016 | NONE | | |
| WO 2006091636 | A2 | 31-08-2006 | US | 2006200034 A1 | 07-09-2006 |
| | | | US | 2006200035 A1 | 07-09-2006 |
| | | | US | 2010195770 A1 | 05-08-2010 |
| | | | US | 2011313760 A1 | 22-12-2011 |
| | | | US | 2013282339 A1 | 24-10-2013 |
| | | | US | 2016087603 A1 | 24-03-2016 |
| | | | WO | 2006091636 A2 | 31-08-2006 |
| WO 2014068436 | A1 | 08-05-2014 | CN | 104755018 A | 01-07-2015 |
| | | | EP | 2914163 A1 | 09-09-2015 |
| | | | JP | 2015533325 A | 24-11-2015 |
| | | | RU | 2015118148 A | 27-12-2016 |
| | | | US | 2015236740 A1 | 20-08-2015 |
| | | | WO | 2014068436 A1 | 08-05-2014 |
| WO 2015049150 | A1 | 09-04-2015 | CN | 105792734 A | 20-07-2016 |
| | | | EP | 3052008 A1 | 10-08-2016 |
| | | | JP | 6194105 B2 | 06-09-2017 |
| | | | JP | 2016538005 A | 08-12-2016 |
| | | | US | 2016220128 A1 | 04-08-2016 |
| | | | WO | 2015049150 A1 | 09-04-2015 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **VIOLA, P. ; JONES, M.J.** Robust real-time object detection. *Proc. of IEEE workshop on statistical and computational theories of vision,* 13 July 2001